Europäisches Patentamt

European Patent Office

Office européen des brevets

(19)

(11) Numéro de publication : **0 031 265**
**B1**

(12) FASCICULE DE BREVET EUROPÉEN

(45) Date de publication du fascicule du brevet :
24.11.82

(21) Numéro de dépôt : 80401699.6

(22) Date de dépôt : 28.11.80

(51) Int. Cl.³ : **C 07 D495/04** // (C07D495/04,
333/00, 221/00)

(54) Procédé de préparation catalytique de la thiéno (3,2-c)pyridine.

(30) Priorité : 20.12.79 FR 7931247

(43) Date de publication de la demande :
01.07.81 (Bulletin 81/26)

(45) Mention de la délivrance du brevet :
24.11.82 Bulletin 82/47

(84) Etats contractants désignés :
DE NL SE

(56) Documents cités :
R.C. ELDERFIELD : « Heterocyclic Compounds »,
vol. 4, 1952 John Wiley NEW YORK (US) « Quinoline, Isoquinoline and their Benzo Derivatives »
pages 399-401

(73) Titulaire : SANOFI, Société dite:
40, Avenue George V
F-75008 Paris (FR)

(72) Inventeur : Braye, Emile
Le Puntis La Gardelle/Lèze
F-31190 Auterive (FR)
Inventeur : Ollivier, Jean-Marie
Croix de Duzy
F-64260 Arudy (FR)

(74) Mandataire : Polus, Camille et al.
c/o Cabinet Lavoix 2, Place d'Estienne d'Orves
F-75441 Paris Cedex 09 (FR)

# Procédé de préparation catalytique de la thiéno[3,2-c]pyridine

La présente invention est relative à un procédé de préparation de la thiéno[3,2-c]pyridine par déshydrogénation catalytique.

Plusieurs procédés conduisant aux dérivés de thiéno[3,2-c]pyridine et/ou [2,3-c]pyridine ont déjà été décrits dans la littérature mais ils sont, soit difficilement transposables à l'échelle industrielle et/ou trop onéreux, soit inapplicables à la préparation de certains dérivés substitués sur le système cyclique. Ainsi, les voies d'accès mentionnées par W. HERTZ et L. TSAI (J. Amer. Chem. Soc., 1953, 75, 5122) ; par C. HANSCH, W. CARPENTER et J. TODD (J. Org. Chem., 1958, 23, 1924) ; par L.H. KLEMM, J. SHABTOY, D.R. McCOY et W.K. KIANG (J. Het. Chem., 1968, 5883 et ibid, 1969, 6813) ; par S. GRONOWITZ et E. SANDBERG (Ark. Kemi, 1970, 32, 217) ; par F. ELOY et A. DERYCKERE (Bull. Soc. Chim. Belges, 1970, 79, 301) ; par J.P. MAFFRAND et F. ELOY (J. Het, Chem., 1976, 13, 1347) ; par A. HEYMES et J.P. MAFFRAND (brevet français 23 12 498) ; par J.P. MAFFRAND (demande de brevet en FRANCE No. 23 95 271) ; ou par R. BOIGEGRAIN et J.P. MAFFRAND (demande de brevet en FRANCE No. 24 24 278), présentent un ou plusieurs des inconvénients cités plus haut.

De plus, aucun des procédés mentionnés ci-dessus ne décrit de réaction de déshydrogénation catalytique du type de la présente invention.

On connaît également un procédé catalytique de déshydrogénation de tétrahydro- et 3,4-dihydro-isoquinoléines (R.C. ELDERFIELD : Heterocyclic Compounds, vol. 4, 1952, John WILEY, New-York, p. 399-341, Catalytic dehydrogenation), mais ce procédé s'applique à des matières premières différentes. Or, une des caractéristiques essentielles des réactions catalytiques est leur spécificité et de plus les catalyseurs de ce document sont différents de ceux de la présente invention.

La présente invention a pour but de fournir un procédé de synthèse peu onéreux permettant d'obtenir avec de bons rendements la thiéno[3,2-c]pyridine qui est un intermédiaire important dans l'industrie chimique et pharmaceutique, en particulier pour la préparation de thiéno-pyridines présentant une activité importante contre l'agrégation plaquettaire.

La présente invention a ainsi pour objet un procédé de préparation de la thiéno[3,2-c]pyridine, caractérisé en ce qu'on soumet une tétrahydro-4,5,6 thiéno[3,2-c]pyridine à une déshydrogénation catalytique, en phase gazeuse, sur un catalyseur à base d'un ou de plusieurs métaux ou de leurs oxydes, choisi parmi le chrome, le nickel, le molybdène, le cobalt et le tungstène éventuellement en association avec du magnésium, du sodium et du fer, déposés sur un support inerte.

Cette réaction est effectuée en phase gazeuse, éventuellement en présence d'un diluant inerte, à une température comprise entre 350 et 600 °C, et de préférence entre 450 et 500 °C.

La tétrahydro-4,5,6,7 thiéno[3,2-c]pyridine de départ peut être préparée par des procédés connus qui ont notamment été décrits par la demanderesse dans ses demandes de brevet français No. 2 300 090, 2 319 642 et 2 397 417.

La réaction catalytique en phase gazeuse est réalisée sous une pression partielle ou totale de tétrahydrothiénopyridine de 5 à 1 000 torrs (6,65 à $1 333 \times 10^2$ Pa), et de préférence comprise entre 50 et 150 torrs (66,5 et $199,5 \times 10^2$ Pa).

La phase gazeuse de tétrahydrothiénopyridine est obtenue par des moyens classiques qui sont, par exemple, l'évaporation du composé en phase liquide maintenu à une température et une pression réglées (notamment sous pression réduite) ou dans une zone de préchauffage en présence d'un courant de gaz inerte dont le débit et la pression sont réglés de façon telle que l'on obtienne la pression partielle désirée de tétrahydrothiénopyridine. Dans le cas d'une dilution par un gaz inerte, on opère avantageusement à une pression totale équivalente à la pression atmosphérique régnante.

Les gaz inertes utilisables sont, par exemple, l'azote, l'hélium, le néon ou l'argon.

La réaction est conduite par passage en continu de la phase gazeuse à une vitesse appropriée sur le catalyseur afin d'obtenir un temps de contact compris entre 0,1 seconde et 10 minutes, et de préférence entre 1 et 10 secondes.

Les trois paramètres définis précédemment, à savoir température, pression partielle et temps de contact, sont liés par des lois cinétiques et thermodynamiques bien connues qui permettent un réglage de la réaction. Ainsi, par exemple, aux basses températures correspondent les temps de contact les plus longs.

Les catalyseurs utilisés pour cette déshydrogénation sont soit à base d'un ou de plusieurs métaux, soit à base d'oxydes de ces métaux choisis parmi le chrome, le nickel, le molybdène, le tungstène et le cobalt, éventuellement en association avec du magnésium, du sodium et/ou du fer. Les métaux ou leurs oxydes sont déposés sur des supports inertes appropriés classiques tels que l'alumine, le charbon actif, la silice, le kieselguhr, etc.

Une première gamme de catalyseurs est à base d'oxyde de chrome ($Cr_2O_3$) dont la quantité déposée sur le support inerte tel que l'alumine peut varier de 1 à 33 % en poids, et de préférence de 15 à 25 % ; l'activité catalytique peut être augmentée par addition de faibles quantités d'oxyde de magnésium, d'oxydes de sodium et/ou d'oxyde de fer.

Une autre gamme de catalyseurs utilisables pour cette déshydrogénation est à base d'oxyde de nickel ou de cobalt/oxyde de molybdène. Le support préféré est l'alumine. La teneur en oxyde de nickel ou de cobalt peut varier entre 1 et 5 % en poids par rapport au support et celle de l'oxyde de molybdène entre 5 et 20 %.

Une troisième gamme de catalyseurs contient du nickel et du tungstène dont les teneurs varient respectivement entre 3 et 10 % et entre 5 et 25 %. Le support préféré est l'alumine, mais la silice, le kieselguhr ou le charbon actif peuvent aussi être utilisés.

On donnera ci-après, à titre d'illustration, un mode de mise en œuvre du procédé de la présente invention.

Exemple 1

La réaction de déshydrogénation catalytique selon l'invention est réalisée dans un réacteur tubulaire vertical placé dans un four permettant d'atteindre des températures supérieures à 600 °C. Le réacteur comporte trois parties. La partie inférieure est constituée d'anneaux Raschig. La partie médiane contient 40 ml (36 g) de catalyseur contenant 20 % d'oxyde de chrome, 0,5 % d'oxyde de sodium Na$_2$O et 0,05 % d'oxyde ferrique, le support étant de la gamma-alumine dont la surface de contact, est de 150 m$^2$/g. Au-dessus du lit catalytique on dépose une couche de billes de Pyrex®. Cette partie supérieure du réacteur sert à évaporer la tétrahydrothiénopyridine et à amener les vapeurs à la température de réaction choisie et qui est aussi celle du catalyseur. Une série de thermo-couples permet de vérifier la température tout au long du réacteur. La partie inférieure du four est suivie d'un piège maintenu à une température adéquate pour condenser les vapeurs sortantes tandis que la partie supérieure du réacteur est reliée à une conduite par où on introduit un courant continu et connu d'azote et à une pompe doseuse qui délivre la tétrahydrothiénopyridine liquide.

Le catalyseur est préalablement activé par passage, pendant 18 heures, d'un courant d'azote à travers le réacteur dont le lit catalytique est réglé à 470 °C.

La réaction de déshydrogénation proprement dite est réalisée en réglant le débit de la pompe doseuse de tétrahydrothiénopyridine liquide à 13,9 g/h et le débit d'azote à 18 l/h (à 20 °C), sous pression normale. Dans ces conditions, le temps de contact est d'environ 2,5 secondes et la vitesse spatiale de 1 300/l/l/h.

Le condensat recueilli cristallise en majeure partie à la température ambiante dans le piège ; une partie des vapeurs est entraînée par l'azote. Le condensat est redistillé pour éliminer une faible quantité de produits colorés formés.

Une analyse du distillat par chromatographie en phase gazeuse indique un taux de conversion de 93 % et une sélectivité de 91,6 % environ. Les cristaux obtenus par refroidissement fondent entre 46,5 et 50 °C. Les spectres IR et RMN obtenus à partir des cristaux sont identiques à ceux d'un étalon de thiéno[3,2-c]pyridine.

Exemple 2

Dans un réacteur horizontal rempli de façon semblable à celui de l'exemple 1 et contenant 40 ml de catalyseur composé de 6 % de nickel, 19 % de tungstène et 75 % d'alumine, on fait passer des vapeurs de tétrahydro-4,5,6,7 thiéno[3,2-c]pyridine à raison de 13,9 g/h sous une pression de 90 mm Hg absolus (120 × 10$^2$ Pa). Pour ce faire, la sortie du piège est reliée à une pompe à vide par l'intermédiaire d'une vanne pointeau de précision, réglée pour obtenir la pression désirée dans le réacteur. On peut aussi utiliser un manostat pour obtenir ce même résultat. Dans ces conditions, le taux de récupération est très bon ; la sélectivité et le taux de conversion sont analogues à ceux de l'exemple 1.

Revendications

1. Procédé de préparation de la thiéno[3,2-c]pyridine, caractérisé en ce qu'on soumet une tétrahydro-4,5,6,7 thiéno[3,2-c]pyridine à une déshydrogénation catalytique, en phase gazeuse, à une température de 350 à 600 °C sur un catalyseur à base d'un ou de plusieurs métaux ou de leurs oxydes, choisis parmi le chrome, le nickel, le molybdène, le cobalt et le tungstène, éventuellement en association avec un ou plusieurs métaux ou leurs oxydes choisis parmi le magnésium, le sodium et le fer, déposés sur un support inerte.

2. Procédé de préparation de la thiéno[3,2-c]pyridine selon la revendication 1, caractérisé en ce que le catalyseur est à base d'oxydes métalliques, choisis parmi les oxydes de chrome, de nickel, de molybdène, de cobalt et de tungstène, éventuellement en association avec un ou plusieurs oxydes métalliques choisis parmi les oxydes de magnésium, de sodium et de fer, déposés sur un support inerte.

3. Procédé selon la revendication 1 ou 2, caractérisé en ce que la réaction de déshydrogénation catalytique est réalisée à une température de 450 à 500 °C.

4. Procédé selon l'une quelconque des revendications précédentes, caractérisé en ce que la réaction est réalisée sous une pression partielle ou totale de tétrahydro-4,5,6,7 thiéno[3,2-c]pyridine de 5 à 1 000 torrs (6,65 à 1 333 × 10$^2$ Pa), et un temps de contact avec le catalyseur de 0,1 seconde à 10 minutes.

5. Procédé selon la revendication 4, caractérisé en ce que la pression partielle ou totale de tétrahydro-4,5,6,7 thiéno[3,2-c]pyridine est de 50 à 150 torrs et le temps de contact avec le catalyseur de 1 à 10 secondes.

6. Procédé selon la revendication 3 ou 4, caractérisé en ce que la phase gazeuse contient un diluant inerte tel que l'azote, l'hélium, le néon ou l'argon.

7. Procédé selon l'une quelconque des revendications précédentes, caractérisé en ce que le support inerte est choisi parmi l'alumine, le charbon actif, la silice et le kieselguhr.

8. Procédé selon l'une quelconque des revendications précédentes, caractérisé en ce que le catalyseur est constitué d'oxyde de chrome

(Cr$_2$O$_3$) déposé à raison de 1 à 33 % en poids sur un support d'alumine et contient éventuellement de faibles proportions d'oxydes de magnésium, de sodium et/ou de fer.

9. Procédé selon l'une quelconque des revendications 1 à 7, caractérisé en ce que le catalyseur est constitué d'oxyde de nickel ou de cobalt, à raison de 1 à 5 % en poids et d'oxyde de molybdène à raison de 5 à 20 % en poids, déposés sur un support d'alumine.

10. Procédé selon l'une quelconque des revendications 1 à 7, caractérisé en ce que le catalyseur est constitué de 3 à 10 % de nickel et de 5 à 25 % de tungstène déposés sur un support d'alumine.

**Claims**

1. Process for the preparation of thieno[3,2-c]pyridine, wherein 4,5,6,7-tetrahydrothieno[3,2-c]pyridine is catalytically dehydrogenated in the gaseous phase at a temperature of from 350 to 600 °C using a catalyst comprising at least one metal and/or metal oxide, said metal being selected from chromium, nickel, molybdenum, cobalt and tungsten, optionally in association with at least one metal and/or metal oxide, said metal being selected from magnesium, sodium and iron, the catalyst being deposited on an inert carrier.

2. Process for the preparation of thieno[3,2-c]pyridine according to claim 1, wherein the catalyst comprises at least one metal oxide, said metal being selected from chromium, nickel, molybdenum, cobalt and tungsten, optionally in association with at least a metal oxide, said metal being selected from magnesium, sodium and iron, the catalyst being deposited on an inert carrier.

3. Process according to claim 1 or 2, wherein the catalytic dehydrogenation reaction is carried out at a temperature of from 450 to 500 °C.

4. Process according to any one of the preceding claims, wherein the reaction is carried out at a partial or total pressure of 4,5,6,7-tetrahydrothieno[3,2-c]pyridine of from 5 to 1 000 torrs (6,65 to 1 333 × 10$^2$ Pa), and a contact time with the catalyst from 0,1 second to 10 minutes.

5. Process according to claim 4, wherein the partial or total pressure of the 4,5,6,7-tetrahydrothieno[3,2-c]pyridine is from 50 to 150 torrs, and the contact time with the catalyst is from 1 to 10 seconds.

6. Process according to claim 3 or 4, wherein the gaseous phase contains an inert diluent such as nitrogen, helium, neon or argon.

7. Process according to any one of the preceding claims, wherein the inert carrier is selected from alumina, active charcoal, silica and kieselguhr.

8. Process according to any one of the preceding claims, wherein the catalyst comprises chromium oxide (Cr$_2$O$_3$) deposited in an amount of from 1 to 33 % by weight on an alumina carrier and optionally contains a minor amount of magnesium oxide and/or sodium oxide and/or iron oxide.

9. Process according to any one of claims 1 to 7, wherein the catalyst comprises nickel or cobalt oxide in an amount of from 1 to 5 % by weight and molybdenum oxide in an amount of from 5 to 20 % by weight, deposited on an alumina carrier.

10. Process according to any one of claims 1 to 7, wherein the catalyst comprises 3 to 10 % by weight of nickel and 5 to 25 % by weight of tungsten, deposited on an alumina carrier.

**Ansprüche**

1. Verfahren zur Herstellung von Thieno[3,2-c]pyridin, dadurch gekennzeichnet, daß man ein 4,5,6,7-Tetrahydrothieno[3,2-c]pyridin in der Gasphase bei einer Temperatur von 350 bis 600 °C mit einem Katalysator katalytisch dehydriert, wobei ein Katalysator auf der Basis von einem oder mehreren der Metalle Chrom, Nickel, Molybdän, Kobalt oder Wolfram oder deren Oxiden, gegebenenfalls zusammen mit einem oder mehreren der Metalle Magnesium, Natrium oder Eisen oder deren Oxiden, auf einem inerten Träger verwendet wird.

2. Verfahren zur Herstellung des Thieno[3,2-c]pyridin, nach Anspruch 1, dadurch gekennzeichnet, daß als Katalysator auf Metalloxidbasis Oxide der Metalle Chrom, Nickel, Molybdän, Kobalt oder Wolfram, gegebenenfalls zusammen mit einem oder mehreren Oxiden der Metalle Magnesium, Natrium oder Eisen auf einem inerten Träger verwendet werden.

3. Verfahren nach Anspruch 1 oder 2, dadurch gekennzeichnet, daß die katalytische Dehydrierung bei einer Temperatur von 450 bis 500 °C durchgeführt wird.

4. Verfahren nach einem der vorhergehenden Ansprüche, dadurch gekennzeichnet, daß die Reaktion unter einem Teil- oder Gesamtdruck des 4,5,6,7-Tetrahydrothieno[3,2-c]pyridin von 5 bis 1 000 Torr (6,65 bis 1 333 × 10$^2$ Pa) und einer Kontaktzeit mit den Katalysator von 0,1 Sekunden bis 10 Minuten durchgeführt wird.

5. Verfahren nach Anspruch 4, dadurch gekennzeichnet, daß der Teil- oder Gesamtdruck des 4,5,6,7-Tetrahydrothieno[3,2-c]pyridin 50 bis 150 Torr und die Kontaktzeit mit dem Katalysator 1 bis 10 Sekunden betragen.

6. Verfahren nach Anspruch 3 oder 4, dadurch gekennzeichnet, daß die Gasphase ein inertes Verdünnungsmittel wie Stickstoff, Helium, Neon oder Argon enthält.

7. Verfahren nach einem der vorhergehenden Ansprüche, dadurch gekennzeichnet, daß der inerte Träger Aluminiumoxid, Aktivkohle, Siliziumoxid oder Kieselgur ist.

8. Verfahren nach einem der vorhergehenden Ansprüche, dadurch gekennzeichnet, daß der Katalysator aus Chromoxid (Cr$_2$O$_3$), das in Mengen von 1 bis 33 Gewichtsprozent auf einem Aluminiumoxidträger aufgebraucht ist, besteht, und gegebenenfalls geringe Mengen an Oxiden des Magnesiums, Natriums und/oder Eisens enthält.

9. Verfahren nach einem der vorhergehenden Ansprüche 1 bis 7, dadurch gekennzeichnet, daß der Katalysator aus Nickel- oder Kobaltoxid in Mengen von 1 bis 5 Gewichtsprozent oder aus Molybdänoxid in Mengen von 5 bis 20 Gewichtsprozent auf einem Aluminiumoxidträger besteht.

10. Verfahren nach einem der Ansprüche 1 bis 7, dadurch gekennzeichnet, daß der Katalysator aus 3 bis 10 % Nickel und 5 bis 25 % Wolfram auf einem Aluminiumoxidträger besteht.